# EUROPEAN PATENT APPLICATION

(11) **EP 4 248 841 A1**
(43) Date of publication of application: **27.09.2023**
(21) Application number: 22163608.7
(22) Date of filing: 22.03.2022
(51) Int. Cl.: A61B 5/00, A61B 5/024, H04B 5/00

(54) **WIRELESSLY POWERING WEARABLE MEDICAL DEVICES**

(71) Applicant: Koninklijke Philips N.V., 5656 AG Eindhoven (NL); Nederlandse Organisatie voor toegepast-natuurwetenschappelijk Onderzoek TNO, 2595 DA 's-Gravenhage (NL)
(72) Inventor: Gelissen, Jozef Hubertus, 5656 AG Eindhoven (NL); Rentrop, Cornelis Hermanus Arnoldus, 2595 DA s-Gravenhage (NL)
(74) Representative: Philips Intellectual Property & Standards

(57) **Abstract**

The present disclosure describes various systems (100) of providing wireless power to one or more wearable medical devices. Specifically, the systems are directed to novel power solutions that enable the use smaller and more comfortable wearable medical devices. According to a first embodiment of the present disclosure, satellite devices (104) are disclosed wherein each satellite device (104) wirelessly receives power from a primary wearable medical device (102). According to a second embodiment of the present disclosure, one or more wearable medical devices (102, 104) are wirelessly supplied power via a nearby power supply source.

## Description

### Field of the Disclosure

The present disclosure is directed generally to powering wearable medical devices, and more specifically, to systems of wirelessly powering wearable medical devices.

### Background

In conventional wearable devices, including wearable medical devices, power requirements can be a significant issue for long-term use. Typically, increasing battery capacity increases the size (or bulkiness) of a wearable device, which may impair comfort and usability for the user of the wearable device. For wearable medical patches, a decrease in comfort is one of the main causes of early skin detachment.

### Summary of the Disclosure

The present disclosure is directed generally to systems for taking bio-potential measurements, and more specifically, to systems of adaptively taking bio-potential measurements within a changing environment. The invention is defined by the independent claims. The dependent claims define advantageous embodiments.

According to an embodiment of the present disclosure, a wireless power system for wearable medical devices is provided. The wireless power system can comprise: a first wearable medical device comprising at least a first sensor for measuring at least a first signal associated with a patient wearing the first wearable medical device, a wireless power source, and a first controller configured to operate the first wearable medical device; and a second wearable medical device comprising at least a second sensor for measuring at least a second signal associated with the patient wearing the second wearable medical device, a wireless power receiver, and a second controller configured to operate the second wearable medical device. In particular, the wireless power source of the first wearable medical device may be configured to supply wireless power to at least the wireless power receiver of the second wearable medical device.

In an aspect, the first controller may comprise at least a first processor, a first memory, and instructions stored in the first memory that, when executed by at least the first processor, may cause the first controller to perform one or more of the following: operate the wireless power source to generate a wireless power supply; measure, via at least the first sensor, at least the first signal associated with the patient wearing the first wearable medical device; store, in the first memory, at least the first measured signal; and transmit, via an interface bus of the first controller, at least the first measured signal.

In an aspect, the second controller may comprise at least a second processor, a second memory, and instructions stored in the second memory that, when executed by at least the second processor, may cause the second controller to perform one or more of the following: measure, via at least the second sensor, at least the second signal associated with the patient wearing the second wearable medical device; store, in the second memory, at least the second measured signal; transmit, via an interface bus of the second controller, at least the second measured signal.

In an aspect, the memory of the first controller may further store instructions that, when executed by at least the first processor, may cause the first controller to perform one or more of the following: receive, via the interface bus of the first controller, at least the second measured signal from at least the second wearable medical device; store, in the memory of the first controller, at least the second measured signal received from at least the second wearable medical device; and transmit, via the interface bus of the first controller, at least the first and second measured signals.

In an aspect, the wireless power source may comprise a transmitter antenna configured to generate an electromagnetic field suitable for inductively transmitting power to the wireless power receiver, and the wireless power receiver may comprise an inductive receiver antenna configured to generate a current under the influence of the electromagnetic field generated by the wireless power source.

In an aspect, the second wearable medical device may further comprise a power storage device configured to store power wirelessly provided to the wireless power receiver from the wireless power source.

In an aspect, the first sensor of the first wearable medical device may comprise at least one of the following: an electrode; a pulse oximeter; an accelerometer; a gyroscope; a temperature sensor; a microphone; an ultrasound transducer; a pressure transducer; and a fluid sensor; and the second sensor of the second wearable medical device may comprise at least one of the following: an electrode; a pulse oximeter; an accelerometer; a gyroscope; a temperature sensor; a microphone; an ultrasound transducer; a pressure transducer; and a fluid sensor.

In an aspect, the first wearable medical device may further comprise: a wireless power receiver comprising an inductive receiver antenna configured to generate a current under the influence of an electromagnetic field generated by an external power source; and a power storage device configured to store power provided to the first wearable medical device.

In an aspect, at least one of the power storage devices of the first wearable medical device and the power storage device of the second wearable medical device may be a rechargeable power storage device.

In an aspect, at least one of the power storage devices of the first wearable medical device and the power storage device of the second wearable medical device may be a disposable power storage device.

In an aspect, the first wearable medical device may be worn at a first location of the patient, the second wearable medical device may be worn at a second location of the patient, and the first and second wearable medical devices may not be in physical contact with one another.

In an aspect, the second location of the patient is an underarm region of the patient.

In an aspect, the wireless power system may further comprise at least a third wearable medical device comprising at least a third sensor for measuring at least a third signal associated with the patient wearing the third wearable medical device, a wireless power receiver, and a third controller configured to operate the third wearable medical device. In particular, the wireless power source of the first wearable medical device may be further configured to supply wireless power to at least the wireless power receiver of the second and third wearable medical devices.

According to another embodiment of the present disclosure, a wireless power system for providing power to one or more wearable medical devices within a wireless power-enabled environment is provided, wherein the wireless power system comprises: a wireless power source comprising a transmitter antenna configured to generate an electromagnetic field suitable for inductively transmitting power to one or more wearable medical devices. In particular, each wearable medical device of the one or more wearable medical devices may include a wireless power receiver comprising an inductive receiver antenna configured to generate a current under the influence of the electromagnetic field generated by the wireless power source.

In an aspect, the one or more wearable medical devices may include at least a first wearable medical device and at least a second wearable medical device. The first wearable medical device may further comprise at least a first sensor for measuring at least a first signal associated with a patient wearing the first wearable medical device, a wireless power source, and a first controller configured to operate the first wearable medical device, and the second wearable medical device may further comprise at least a second sensor for measuring at least a second signal associated with the patient wearing the second wearable medical device, and a second controller configured to operate the second wearable medical device. In particular, the wireless power source of the first wearable medical device is configured to supply wireless power to at least the wireless power receiver of the second wearable medical device.

These and other aspects of the various embodiments will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1A is a block diagram of a wireless power system illustrated according to aspects of the present disclosure.
FIG. 1B is a block diagram of a controller for use in one or more wearable medical devices illustrated according to aspects of the present disclosure.

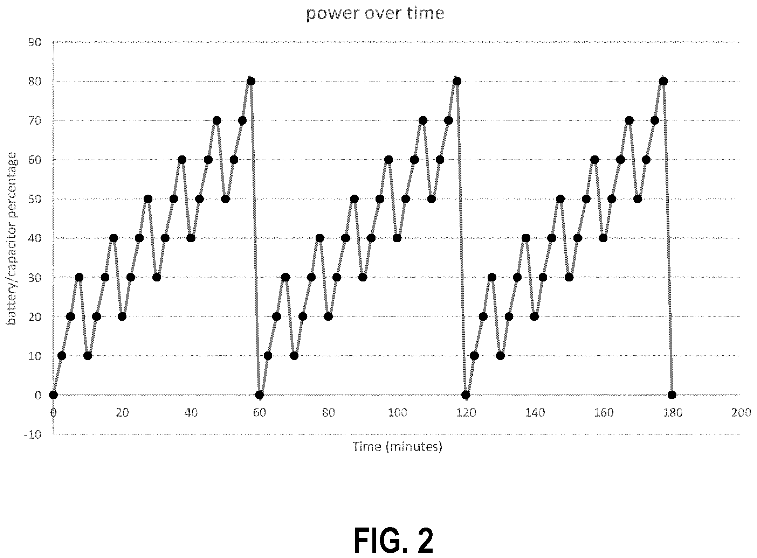

FIG. 2 is a diagram of a wireless power system for providing power to one or more wearable medical devices within a wireless power-enable environment illustrated according to aspects of the present disclosure.
FIG. 3 is a graph illustrating the power consumption of a wearable medical device according to aspects of the present disclosure.
FIG. 4 is an illustration showing a patient using a wireless power system illustrated according to aspects of the present disclosure.
FIG. 5 is an illustration showing the placement of a wearable medical device according to aspects of the present disclosure.

### Detailed Description of Embodiments

The present disclosure describes wireless power systems of wirelessly powering wearable medical devices. In the healthcare setting, a patient may be outfitted with a number of medical devices, including both wired and wireless devices, for various purposes, such as monitoring the patient's vital signs, measuring certain physiological signals from the patient, and providing treatment to the patient. Unless a healthcare specialist is constantly applying and removing these wearable medical devices, the patient may be required to wear such devices over long periods of time (i.e., days to weeks, or longer). However, requiring patients to wear certain devices over longer periods of time often limits the patient's ability to get up and move around (i.e., decreases patient mobility). Additionally, in order to obtain clinically relevant data, these wearable medical devices may be placed on the patient's body in awkward or uncomfortable locations, which further exacerbates the lack of comfortability and/or wearability of the device. For example, when measuring a patient's temperature, measurements can be taken at a variety of locations on the patient, but not all of those locations will yield an accurate measurement of the patient's core body temperature. One location where a clinically relevant measurement of the patient's core body temperature can be taken is underneath the patient's arm on the patient's armpit, but wearing a large medical device located in the armpit is uncomfortable and leads to detachment from the skin under simple, day-to-day movement. Moreover, when a patient is wearing a medical device for longer periods of time, a healthcare specialist may not want and/or need to take measurements of the patient continuously. For example, if a patient just walked up a flight of stairs, taking measurements of the patient's respiration rate, blood pressure, heart rate, and the like may not yield clinically relevant results. As described herein, the wireless power systems allow for the use of small and/or miniaturized wearable medical devices that can be ideally placed and selectively powered to collect clinically relevant measurements, thereby improving comfortability of the wearable device and overall patient experience.

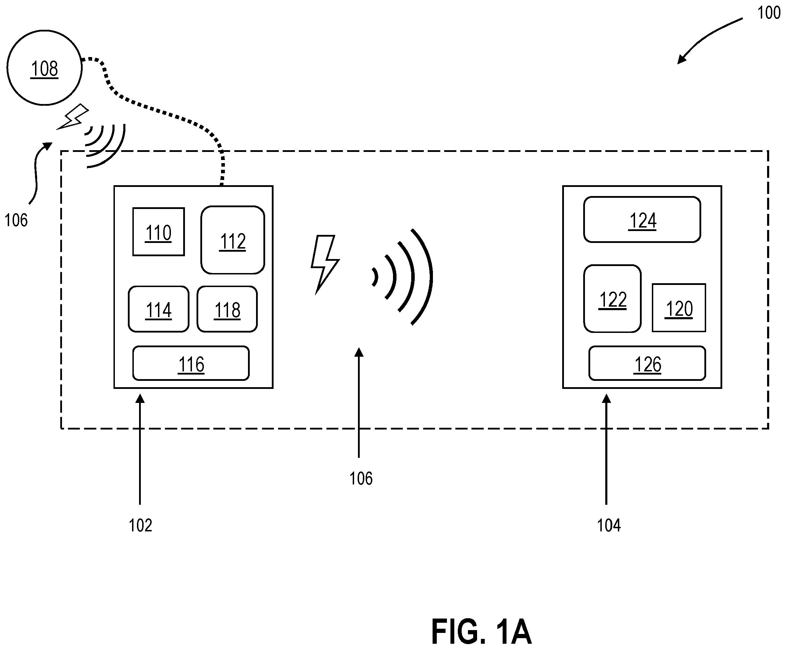

With reference to FIG. 1A, a wireless power system 100 for wearable medical devices is illustrated according to aspects of the present disclosure. As shown, the wireless power system 100 can include at least a first wearable medical device 102 and at least a second wearable medical device 104. In embodiments, the first wearable medical device 102 can comprise at least a first sensor 110, a wireless power source 114, and a first controller 112 configured to operate the first wearable medical device 102. In further embodiments, the second wearable medical device 104 can comprise at least a second sensor 120, a wireless power receiver 124, and a second controller 122 configured to operate the second wearable medical device 104.

As illustrated in FIG. 1A, the wireless power source 114 of the first wearable medical device 102 can be configured to supply wireless power 106 to at least the wireless power receiver 124 of at least the second wearable medical device 104. In embodiments, the wireless power source 114 can comprise a transmitter antenna configured to generate an electromagnetic field that is suitable for inductively transmitting power to the wireless power receiver 124 of at least the second wearable medical device 104. As such, the wireless power receiver 124 of at least the second wearable medical device 104 can comprise an inductive receiver antenna configured to produce a current under the influence of the electromagnetic field generated by the wireless power source 114.

In embodiments, at least the first sensor 110 of the first wearable medical device 102 can be a sensor for measuring at least a first signal associated with a patient (e.g., patient 302) that is wearing the first wearable medical device 102. Similarly, at least the second sensor 120 of the second wearable medical device 104 can be a sensor for measuring at least a second signal associated with the patient (e.g., patient 302) that is also wearing the second wearable medical device 104. In embodiments, at least the first signal and at least the second signal correspond to different physiological measurements associated with the patient (e.g., patient 302). As discussed above, at least the first sensor 110 and the second sensor 120 can be configured to measure clinically relevant signals from the patient (e.g., patient 302).

In embodiments, the first sensor(s) 110 of the first wearable medical device 102 can comprise at least one of an electrode, a pulse oximeter, an accelerometer, a gyroscope, a temperature sensor, a microphone, an ultrasound transducer, a pressure transducer, a fluid sensor, or the like. Further, the second sensor(s) 120 of the second wearable medical device 104 can comprise at least one of an electrode, a pulse oximeter, an accelerometer, a gyroscope, a temperature sensor, a microphone, an ultrasound transducer, a pressure transducer, a fluid sensor, or the like.

As such, depending on the type of sensor(s) included in the first and second wearable medical device 102, 104, the signals measured using the first and second sensors 110, 120 can include one or more of: an electrical signal; oxygen level; heart rate; movement; orientation / position; temperature, such as core body temperature; a human-audible sound signal such as respiratory rate or other indication of respiratory function; a sound signal outside the typical range of human hearing (e.g., above 20 kHz, including in the MHz range); a pressure-dependent electrical signal; a signal dependent on the pH of a collected fluid; a signal dependent on one or more other biomarkers (e.g., concentration of Na⁺ ions) of a collected fluid; and the like. In other words, each of at least the first and second wearable medical devices 102, 104 can independently measure physiological parameters, including: action potentials and/or electrical activity within the patient (e.g., patient 302); oxygen levels; movement, orientation, and/or position of the patient (e.g., patient 302); temperature, such as core body temperature; heart rate; soundwaves indicative of respiratory rate and/or function; ultrasound waves; pressures such as blood pressure; biomarkers within fluids such as sweat, including but not limited to pH and Na⁺ concentration; and the like.

In embodiments, the first wearable medical device 102 can comprise one or more of the same sensors or different sensors than the second wearable medical device 104. In an aspect, at least the first sensor 110 can be a different sensor than at least the second sensor 120. In such embodiments, at least the first signal and at least the second signal measured by the first and second wearable medical devices 102, 104, respectively, are different physiological signals. In other aspects, at least the first sensor 110 can include the same sensor as one of the second sensor(s) 120, such that one of the first and second signals measured by the first and second wearable medical devices 102, 104 are the same physiological signal (or type of physiological signal).

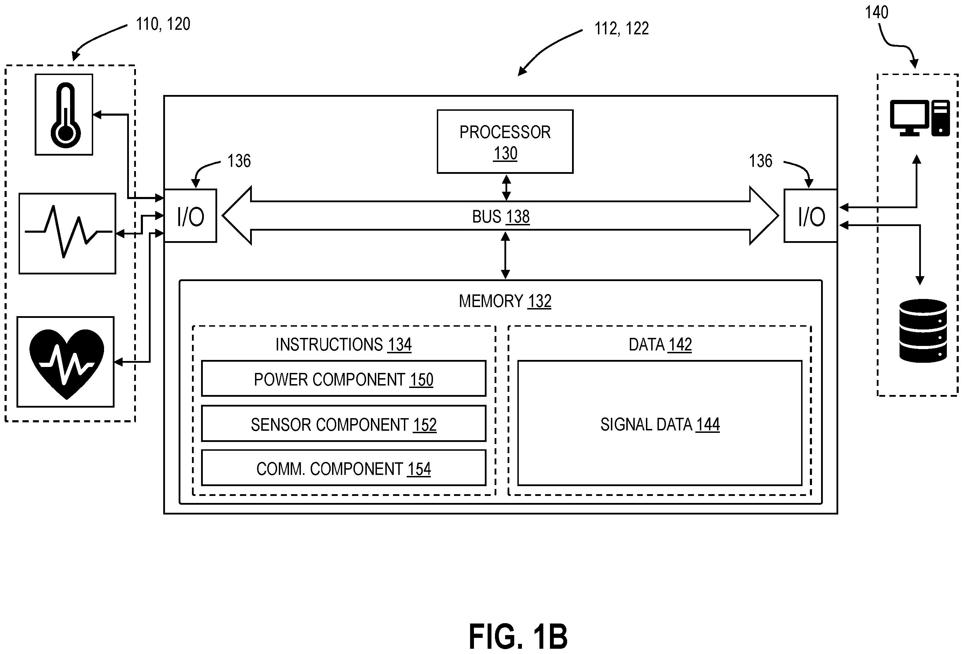

With reference to FIG. 1B, a diagram of the controllers (e.g., controllers 112, 122) of the wearable medical devices (e.g., devices 102, 104) are illustrated in accordance with certain aspects of the present disclosure. As shown, each of the at least first and second controllers 112, 122 can include at least one processor 130 operatively connected to a memory storage device 132 that stores instructions 134 for operating the corresponding wearable medical device 102, 104. The at least one processor 130 can be a general-purpose processor, a microprocessor, or any conventional processor, controller, microcontroller, or state machine. The processor 130 also may be implemented as a combination of computing devices, such as a combination of a digital signal processor (DSP) and a microprocessor, a plurality of microprocessors, one or more microprocessors in conjunction with a DSP core, or any other such configuration. The memory storage device 132 can comprise one or more types of transitory and/or non-transitory machine-readable memory, including but not limited to, RAM, ROM, EPROM, EEPROM, or other optical disk storage, magnetic disk storage or other magnetic storage devices, solid-state storage devices such as flash-based solid-state storage devices (e.g., SD card, micro-SD card, SSD, USB flash drives, etc.), or any other medium that can be used to carry or store desired program code in the form of machine-executable instructions or data structures and which can be accessed by a general purpose, a special purpose, or other machine with a processor. As used here, machine-executable instructions 134 can include, for example, instructions 134 and data 142 which cause a general-purpose computer, special purpose computer, or special purpose processing machines to perform a certain function or group of functions.

In embodiments, each of the controllers 112, 122 can further include an interface bus 136 that comprises one or more components to facilitate communication with peripheral / external devices. For example, and without limitation, the interface bus 136 can include a wired and/or wireless communications interface that allow the controller 112, 122 to send and receive information from the one or more peripheral devices using various communication protocols, such as Wi-Fi, Bluetooth, cellular data networks (e.g., 3G, 4G, 5G, LTE, etc.), or the like. In particular embodiments, the interface bus 136 can include a communications interface that facilitates wireless communication over a private, dedicated spectrum, such as within the 1.4 GHz band.

As shown in FIG. 1B, the interface bus 136 can facilitate communication with the one or more sensors 110, 120 of the wearable medical devices 102, 104, as well as one or more peripheral devices 140. In embodiments, the peripheral devices 140 can include one or more servers, cloud computing devices, backend servers, mobile devices, smartphones, other mobile phones, tablet computers, wearable computing devices, desktop computers, laptop computers, display screens, one or more user input devices such as a keyboard, touchpad, touch screen, mouse or other point device, scroll wheel, click wheel, dial, button, stylus, switch, keypad, microphone, camera, and/or combinations thereof. In particular embodiments, the peripheral devices 140 comprise at least a patient monitor having a display for displaying the signals measured by the sensors 110, 120 of the wireless power system 100. The display can incorporate various image generating technologies, such as a liquid crystal display (LCD), light-emitting diode (LED) including organic light-emitting diodes (OLED), projection system, cathode ray tube (CRT), or the like, together with supporting electronics (e.g., digital-to-analog or analog-to-digital converts, signal processors, or the like). A device such as a touchscreen that functions as both input and output device can also be used.

In embodiments, the interface bus 136 of the controllers 112, 122 can further facilitate wireless communication between one or more of the wearable medical devices 102, 104 themselves. For example, the interface bus 136 of a second controller 122 can facilitate the wireless communication between the second wearable medical device 104 and the first wearable medical device 102. In such embodiments, for example, the second wearable medical device 104 may send and receive data 142 such as signal data 144 (e.g., the second signal measured by at least the second sensor 120 of the second wearable medical device 104) to the first wearable medical device 102.

In addition to transmitting and receiving data 142 such as signal data 144 between two or more wearable medical devices 102, 104, the interface bus 136 of each controller 112, 122 can send and receive other data 142, such as machine-understandable requests, calls, and the like. For example, in particular embodiments, at least the second wearable medical device 104 may need additional power to continue taking measurements and/or to transmit the stored signal data 144, and therefore the second wearable medical device 104 may transmit a request to the first wearable medical device 102 to provide it with additional wireless power (e.g., a request to use its wireless power source 114 to generate an electromagnetic field). Similarly, the first wearable medical device 102 may send and receive similar machine-understandable data 142 from the at least the second wearable medical device 104. For example, in some embodiments, the first wearable medical device 102 may occasionally query at least the second wearable medical device 104 to determine whether it has any signal data 144 and/or other data 142 to transmit, or to determine whether it needs additional power.

As shown in FIG. 1B, the components of the controllers 112, 122, including the processor(s) 130, memory 132, and interface bus 136, can be operatively connected and communicate via a system bus 138. Further, the memory 132 storing instructions 134 can include one or more components that, when executed by the processor(s) 130 of the respectively controller 112, 122, causes the corresponding wearable medical device 102, 104 to perform one or more functions. In general, the instructions 134 of each of the controllers 112, 122 can include at least: a power component 150 responsible for monitoring and maintaining the desired power level of one or more wearable medical device 102, 104 and well as for operating the wireless power source 114 and/or wireless power receiver 124 of the respective devices 102, 104; a sensor component 152 responsible for controlling the one or more sensors 110, 120 of the respective medical devices 102, 104 in order to measure and record the respective signals; and a communications component 154 responsible for sending and receiving data 142 between devices 102, 104 and peripheral devices 140 as well as making requests and queries of such devices.

In particular embodiments, the second memory 132 of the second controller 122 can include instructions 134 that, when executed by at least the second processor 130 of the second controller 122, causes the second wearable medical device 104 to perform one or more of the following: (i) operate the wireless power receiver 124 to receive a wireless power supply 106; (ii) measure, via at least the second sensor 120, at least the second signal associated with the patient (e.g., patient 302) wearing the second wearable medical device 104; (iii) store, in the second memory 132, at least the second measured signals (e.g., as signal data 144); and (iv) transmit, via the interface bus 136 of the second controller 122, at least the second measured signal (e.g., signal data 144).

In further embodiments, the first memory 132 of the first controller 112 can include instructions 134 that, when executed by at least the first processor 130 of the first controller 112, causes the first wearable medical device 102 to perform one or more of the following: (i) operate the wireless power source 114 to generate a wireless power supply 106; (ii) measure, via at least the first sensor 110, at least the first signal associated with the patient (e.g., patient 302) wearing the first wearable medical device 102; (iii) store, in the first memory 132 of the first controller 112, at least the first measured signal (e.g., as signal data 144); and (iv) transmit, via an interface bus 136 of the first controller 112, at least the first measured signal (e.g., signal data 144). The first memory 132 of the first controller 112 can also include instructions 134 that, when executed by at least the first processor 130 of the first controller 112, causes the first wearable medical device 102 to perform one or more of the following: (v) receive, via the interface bus 136 of the first controller 112, at least the second measured signal (e.g., as signal data 144) from at least the second wearable medical device 104; (vi) store, in the first memory 132 of the first controller 112, at least the first and second measured signals (e.g., as signal data 144).

Although only two types of wearable medical devices (i.e., a power-supplying wearable medical device 102, and a satellite wearable medical device 104) have been discussed, the wireless power systems 100 of the present disclosure are not limited to just two such devices 102, 104. For example, the wireless power system 100 of the present disclosure can comprise a plurality of wearable medical devices, wherein the plurality of wearable medical devices includes at least one power-supplying wearable medical device 102 and at least one satellite wearable medical device 104. In embodiments, the wireless power system 100 comprises a power-supplying wearable medical device 102 and at least two satellite wearable medical devices 104 (i.e., a second wearable medical device 104 and a third wearable medical device 104). The third wearable device 104 can comprise at least a third sensor 120 for measuring at least a third signal associated with the patient (e.g., patient 302) wearing the third wearable medical device 104, a wireless power receiver 124, and a third controller 122 configured to operate the third wearable medical device 104 as discussed above. In such embodiments, the wireless power source 114 of the first wearable medical device 102 can be further configured to supply wireless power 106 to at least the wireless power receivers 124 of the second and third wearable medical devices 104. Further, as discussed above, the third sensor can be configured to measure the same or a different signal from the other wearable medical devices 102, 104 and can comprise at least one of the following: an electrode; a pulse oximeter; an accelerometer; a gyroscope; a temperature sensor; a microphone; an ultrasound transducer; a pressure transducer; a fluid sensor; and the like.

Alternatively and/or additionally, the wireless power system 100 of the present disclosure can comprise a plurality of wearable medical devices, wherein the plurality of wearable medical devices includes at least two power-supplying wearable medical devices 102 and at least one satellite wearable medical device 104. In such embodiments, the satellite wearable medical device 104 can be configured to receive a wireless power supply 106 from either of the two power-supplying wearable medical devices 102. These and other combinations of wearable medical devices are specifically contemplated, including wireless power systems (e.g., wireless power system 100) that comprise more than three wearable medical devices 102, 104.

Returning to FIG. 1A, the first wearable medical device 102 (i.e., the power-supplying wearable medical device) can further comprise a power receiver 118 and/or a power storage device 116. In embodiments, the power receiver 118 can be a wireless power receiver 118 and comprise an inductive receiver antenna configured to generate a current under the influence of an electromagnetic field generated by an external power source, such as power source 108. The current generated by the wireless power receiver 118 can be used by the first wearable medical device 102 to power the first wearable medical device 102 and/or operate the wireless power source 114 for powering one or more other devices 104. In alternative embodiments, the power source 108 may be an AC or DC power source and operatively connected to the wearable medical device 102 through a wired connection. In embodiments, the power storage device 116 of the power-supplying wearable device 102 can be configured to store power provided to the device 102, regardless of whether the power is provided wirelessly or not. In further embodiments, at least the second wearable medical device 104 (i.e., satellite device(s) 104) can also include a power storage device 126 configured to store power wirelessly provided to the wireless power receiver 124 of the corresponding satellite device 104 from the wireless power source 114 of at least the first wearable medical device 102. As such, each of the controllers 112, 122 of the wearable medical devices 102, 104 can further include instructions 134 that, when executed by the respective processor(s) 130 of the controllers 112, 122 selectively cause received power to be stored in the respective power storage devices 116, 126. That is, in embodiments, the controllers 112, 122 can include instructions 134 that determines whether to store power in a respective power storage device 116, 126 or to use the existing power (either presently being received / generated or power stored in a storage device 116, 126) in order to operate the corresponding wearable medical device 102, 104.

For example, in embodiments, a satellite device 104 can be configured to determine whether clinically relevant data can be collected (e.g., by querying the power-supplying device 102 or a peripheral device 140), and then request that the power-supplying device 102 provide it wireless power 106 while being operated to measure the clinical-relevant signals. Alternatively, the satellite device 104 may have clinically relevant data 144 stored in a memory 132 of a controller 122 that is ready to be transmitted either to the power-supplying device 102 or to a peripheral device 140, and therefore use such supplied-power 106 to transmit such data 144. In still further embodiments, performance of an energy-intensive task such as transmitting data 144 may be delayed until the device 102, 104 has received enough power to perform such as task.

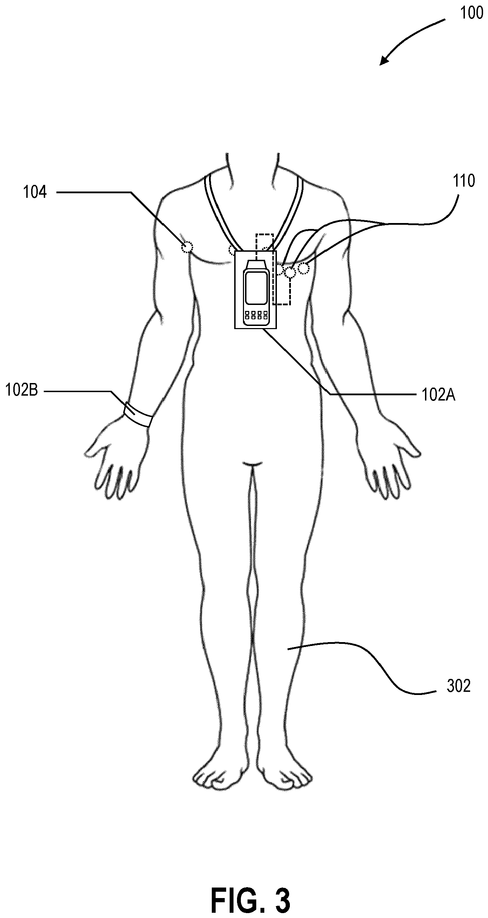

For example, as shown in FIG. 3, a graph showing the energy stores of a satellite device 104 during operation in accordance with one embodiment of the present disclosure. In particular, during the period of time represented in the graph of FIG. 3, the satellite device 104 is continuously receiving power from a power-supplying wearable medical device 102, but at certain intervals the satellite device 104 is operated to take a measurement of a signal associated with the patient (e.g., patient 302) wearing the medical device. Thus, the battery percentage drops from 30% to 10%, then 40% to 20%, then 50% to 30%. In embodiments, the satellite device 104 may selectively perform an energy-intensive task (e.g., transmitting data 144) when the satellite device 104 detects that there is sufficient power to perform an energy-intensive task. As shown in FIG. 3, this occurs at 60 minutes, 120 minutes, and 180 minutes (i.e., once per hour) when the battery storage reaches about 80% capacity.

In this manner, the wearable medical devices 102, 104 of the wireless power systems 100 described herein can be selectively powered and operated to reduce the need for uncomfortable wearable devices that include large / bulky battery packs. It is further contemplated that the wearable medical devices 102, 104 are selectively / not continuously operated or powered.

Turning to FIG. 3, the wearable medical devices 102, 104 of the disclosed wireless power system 100 can be embodied in various types of wearable devices, such as patches, watches or wristbands, or body-mounted devices. In certain embodiments, the power-supplying device 102 can be a device 102A hanging from the patient 302, or can be a wristband or watch-type device 102B, while the satellite device 104 is a patch-type device. In such embodiments, the satellite patch 104 can comprise: (i) a skin adhesive layer for reversibly attaching the device 104 to the skin of the patient 302; (ii) an electronics layer containing the sensor(s) 120, wireless power receiver 124, controller 122, power storage device 126, and the like; (iii) a carrier layer, such as foam or plastic film located between the adhesive layer and the electronics layer where the components of the electronics layer rest upon; and (iv) a top layer enclosure, such as a hard shell, foam, or plastic film that covers and protects the components of the electronics layer.

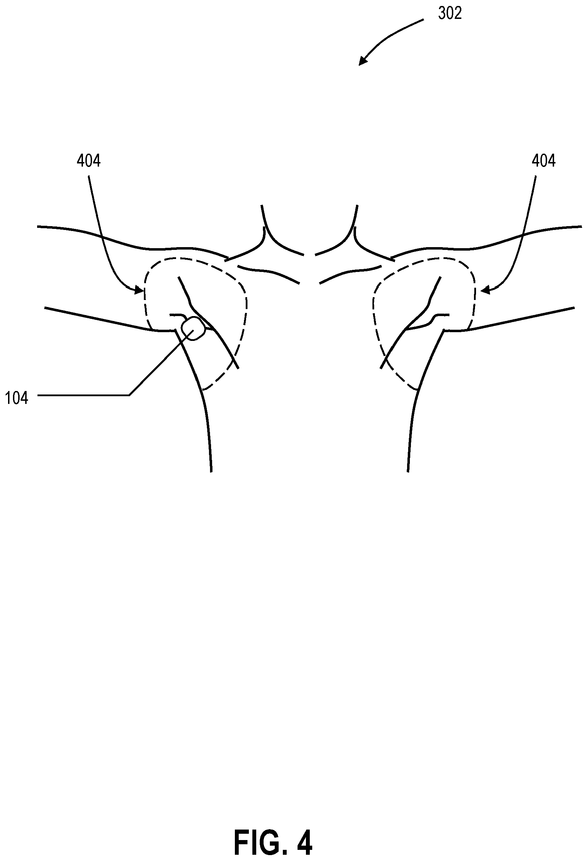

With reference to FIGS. 3 and 4, the wearable medical devices 102, 104 can be worn at different locations of the patient 302 such that the power-supplying device(s) 102 are within a certain proximity of the satellite device 104, within which the satellite device 104 is able to receive power wirelessly from the power-supplying devices(s) 102. In embodiments, at least the first wearable medical device 102 is worn at a first location of the patient 302, at least the second wearable device 104 is worn at a second location of the patient 302, and the devices 102, 104 are not in physical contact with one another. For example, and without limitation, the first location may be adjacent to the chest of the patient 302 or attached to wrist of the patient 302, while the second location can be in a less accessible region of the patient's body 302, such as the underarm / armpit 402, 404 of the patient 302.

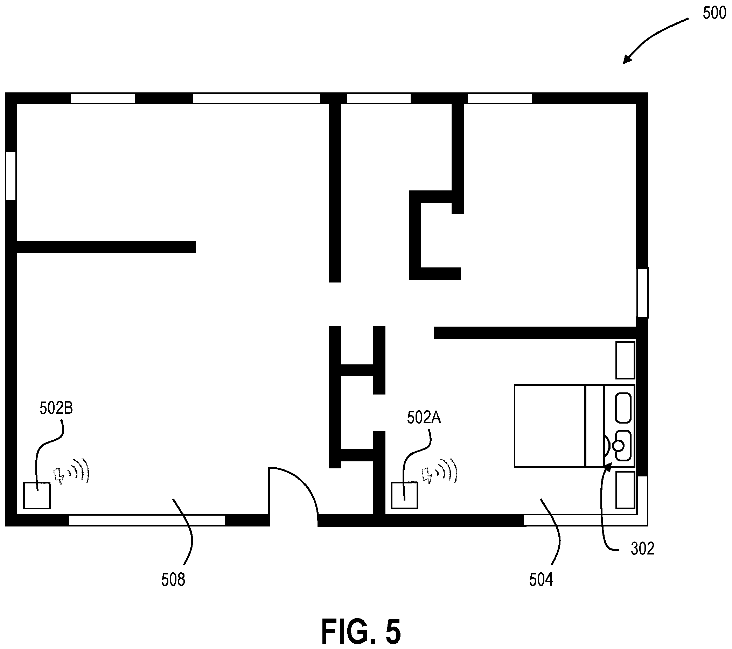

With reference to FIG. 5, a wireless power system 500 for providing power to one or more wearable medical devices 102, 104 within a wireless power-enabled environment 504, 508 is illustrated in accordance with another embodiment of the present disclosure. The wireless power system 500 can comprise at least one wireless power source 502A, 502B comprising a transmitter antenna configured to generate an electromagnetic field suitable for inductively transmitting power to one or more wearable medical devices 102, 104, as discussed above. In an aspect, at least one of the wearable medical devices 102, 104 can include a wireless power receiver 118, 124 that comprise an inductive receiver antenna configured to generate a current under the influence of the electromagnetic field generated by the wireless power source 502A, 502B. In embodiments, both wearable medical devices 102, 104 can be wirelessly powered by the wireless power source 502A, 502B.

In other embodiments, only at least a first wearable medical device 102 comprises a wireless power receiver 118 configured to generate a current under the influence of the electromagnetic field generated by the wireless power source 502A, 502B, while at least the second wearable medical device 104 comprises a wireless power receiver 124 that is configured to generate a current under the influence of an electromagnetic field generated by at least the first wearable medical device 102. As such, in some embodiments, the wireless power source 502A, 502B can generate a first electromagnetic field that is suitable for delivering power wirelessly to only at least the first wearable medical device 102, while the first wearable medical device can generate a different, second electromagnetic field that is suitable for delivering power wirelessly to one or more satellite devices 104.

In embodiments, the wireless power system 500 can comprise one or more wireless power sources 502A, 502B in order to provide power wirelessly within one or more different environments 504, 508. In this manner, a patient 302 may be able to move between different environments, including the one or more wireless power-enabled environments 504, 508, without the system 500 losing the capability to take clinically-relevant measurements from the patient 302, as desired. As used herein, the term "wireless power-enabled environment" refers to a location in which one or more wearable medical devices 102, 104 attached to a patient 302 may wirelessly receive power. For example, the wireless power-enable environment can include: (i) a patient's bed, such as a hospital bed or another bed; (ii) a room, such as a hospital room, bedroom, bathroom, or the like; (iii) a floor of a structure, such as a hospital floor or a story of a home; (iv) a wing of a building; and (v) an entire building or collection of buildings.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "having," "containing," "involving," and the like are to be understood to be open-ended, i.e., to mean including but not limited to.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to block diagrams of apparatus (systems), and computer program products according to examples of the disclosure. It will be understood that each block of the block diagrams, and combinations of blocks in the block diagrams, can be implemented by computer readable program instructions.

The computer readable program instructions can be provided to a processor of a special purpose computer, or other programmable data processing apparatus to produce a machine, such that the instructions, which execute via the processor of the computer or other programmable data processing apparatus, create means for implementing the functions/acts specified in the block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the block diagram block or blocks.

The block diagrams in the Figures illustrate the architecture, functionality, and operation of possible implementations of systems, and computer program products according to various examples of the present disclosure. In this regard, each block in the block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams, and combinations of blocks in the block diagrams, can be implemented by special purpose hardware-based systems that perform the specified functions or acts or carry out combinations of special purpose hardware and computer instructions.

Other implementations are within the scope of the following claims and other claims to which the applicant can be entitled. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material and/or kit described herein. In addition, any combination of two or more such features, systems, articles, materials and/or kits, if such features, systems, articles, materials and/or kits are not mutually inconsistent, is included within the inventive scope of the present disclosure.

## Claims

1. A wireless power system (100) for wearable medical devices, the system comprising:
a first wearable medical device (102) comprising at least a first sensor (110) for measuring at least a first signal associated with a patient (302) wearing the first wearable medical device (102), a wireless power source (114), and a first controller (112) configured to operate the first wearable medical device (102); and
a second wearable medical device (104) comprising at least a second sensor (120) for measuring at least a second signal associated with the patient (302) wearing the second wearable medical device (104), a wireless power receiver (124), and a second controller (122) configured to operate the second wearable medical device (104);
wherein the wireless power source (114) of the first wearable medical device (102) is configured to supply wireless power (106) to at least the wireless power receiver (124) of the second wearable medical device (104).

2. The wireless power system of claim 1, wherein the first controller (112) comprises at least a first processor (130), a first memory (132), and instructions (134) stored in the first memory (132) that, when executed by at least the first processor (130), causes the first controller (112) to perform one or more of the following:
operate the wireless power source (114) to generate a wireless power supply (106);
measure, via at least the first sensor (110), at least the first signal associated with the patient (302) wearing the first wearable medical device (102);
store, in the first memory (132), at least the first measured signal; and
transmit, via an interface bus (136) of the first controller (112), at least the first measured signal.

3. The wireless power system of claim 1, wherein the second controller (122) comprises at least a second processor (130), a second memory (132), and instructions (134) stored in the second memory (132) that, when executed by at least the second processor (130), causes the second controller (122) to perform one or more of the following:
operate the wireless power receiver (124) to receive a wireless power supply (106);
measure, via at least the second sensor (120), at least the second signal associated with the patient (302) wearing the second wearable medical device (104);
store, in the second memory (132), at least the second measured signal;
transmit, via an interface bus (136) of the second controller (122), at least the second measured signal.

4. The wireless power system of claim 3, wherein the memory (132) of the first controller (112) further stores instructions that, when executed by at least the first processor (130), causes the first controller (112) to perform one or more of the following:
receive, via the interface bus (136) of the first controller (112), at least the second measured signal from at least the second wearable medical device (104);
store, in the memory (132) of the first controller (112), at least the second measured signal received from at least the second wearable medical device (104); and
transmit, via the interface bus (136) of the first controller (112), at least the first and second measured signals.

5. The wireless power system of claim 1, wherein the wireless power source (114) comprises a transmitter antenna configured to generate an electromagnetic field suitable for inductively transmitting power to the wireless power receiver (124), and wherein the wireless power receiver (124) comprises an inductive receiver antenna configured to generate a current under the influence of the electromagnetic field generated by the wireless power source (114).

6. The wireless power system of claim 1, wherein the second wearable medical device (104) further comprises a power storage device (126) configured to store power wirelessly provided to the wireless power receiver (124) from the wireless power source (114).

7. The wireless power system of claim 1, wherein the first sensor (110) of the first wearable medical device (102) comprises at least one of the following: an electrode; a pulse oximeter; an accelerometer; a gyroscope; a temperature sensor; a microphone; an ultrasound transducer; a pressure transducer; and a fluid sensor; and
wherein the second sensor (120) of the second wearable medical device (104) comprises at least one of the following: an electrode; a pulse oximeter; an accelerometer; a gyroscope; a temperature sensor; a microphone; an ultrasound transducer; a pressure transducer; and a fluid sensor.

8. The wireless power system of claim 1, wherein the first wearable medical device (102) further comprises:
a wireless power receiver (118) comprising an inductive receiver antenna configured to generate a current under the influence of an electromagnetic field generated by an external power source (108); and
a power storage device (116) configured to store power provided to the first wearable medical device (102).

9. The wireless power system of claim 1, wherein at least one of the power storage devices (116) of the first wearable medical device (102) and the power storage device (126) of the second wearable medical device is a rechargeable power storage device (116, 126).

10. The wireless power system of claim 1, wherein at least one of the power storage devices (116) of the first wearable medical device (102) and the power storage device (126) of the second wearable medical device is a disposable power storage device (116, 126).

11. The wireless power system of claim 1, wherein the first wearable medical device (102) is worn at a first location of the patient (302), the second wearable medical device (104) is worn at a second location of the patient (302), and wherein the first and second wearable medical devices (102, 104) are not in physical contact with one another.

12. The wireless power system of claim 11, wherein the second location of the patient (302) is an underarm region (404) of the patient (302).

13. The wireless power system of claim 1, further comprising:
at least a third wearable medical device (104) comprising at least a third sensor (120) for measuring at least a third signal associated with the patient (302) wearing the third wearable medical device (104), a wireless power receiver (124), and a third controller (122) configured to operate the third wearable medical device (104);
wherein the wireless power source (114) of the first wearable medical device (102) is further configured to supply wireless power (106) to at least the wireless power receiver (124) of the second and third wearable medical devices (104).

14. A wireless power system for providing power to one or more wearable medical devices (102, 104) within a wireless power-enabled environment (204, 208), the wireless power system comprising:
a wireless power source (202A, 202B) comprising a transmitter antenna configured to generate an electromagnetic field suitable for inductively transmitting power to one or more wearable medical devices (102, 104), wherein each wearable medical device (102, 104) of the one ormore wearable medical devices (102,104) includes a wireless power receiver (118, 124) comprising an inductive receiver antenna configured to generate a current under the influence of the electromagnetic field generated by the wireless power source (202A, 202B).

15. The wireless power system of claim 14, wherein the one or more wearable medical devices (102, 104) includes at least a first wearable medical device (102) and at least a second wearable medical device (104),
wherein the first wearable medical device (102) further comprises at least a first sensor (110) for measuring at least a first signal associated with a patient (302) wearing the first wearable medical device (102), a wireless power source (114), and a first controller (112) configured to operate the first wearable medical device (102);
wherein the second wearable medical device (104) further comprises at least a second sensor (120) for measuring at least a second signal associated with the patient (302) wearing the second wearable medical device (104), and a second controller (122) configured to operate the second wearable medical device (104); and
wherein the wireless power source (114) of the first wearable medical device (102) is configured to supply wireless power (106) to at least the wireless power receiver (124) of the second wearable medical device (104).
